# EUROPEAN PATENT APPLICATION

(11) **EP 4 559 846 A1**
(43) Date of publication of application: **28.05.2025**
(21) Application number: 24214827.8
(22) Date of filing: 22.11.2024
(51) Int. Cl.: B65G 47/14, B65B 35/08

(54) **CENTRIFUGAL FEEDER AND TABLET INSPECTION DEVICE COMPRISING SAME**

(30) Priority: 24.11.2023 KR 20230165557; 22.04.2024 KR 20240053672
(71) Applicant: Enclony Inc, Seoul 08382 (KR)
(72) Inventor: LEE, Kyung Ho, 10450 Goyang-si, Gyeonggi-do (KR)
(74) Representative: Schmidt, Steffen J.

(57) **Abstract**

A centrifugal feeder may rapidly supply tablets in a constant position without damage to the tablets during a supply process thereof. The centrifugal feeder includes an inner disk having tablets supplied thereto and rotating with a central axis thereof inclined relative to a vertical direction, an outer disk rotating around while surrounding the inner disk and including a tablet moving surface in which the tablets received from the inner disk are moved, and a pocket guide connected to the outer disk, covering a portion of a tablet moving surface and including a plurality of pockets into which the tablets are received.

## Description

### CROSS-REFERENCE TO RELATED APPLICATION(S)

This application claims benefit of priority to Korean Patent Application No. 10-2023-0165557 and 10-2024-0053672 filed on November 24, 2023 and April 22, 2024 in the Korean Intellectual Property Office, the disclosure of which is incorporated herein by reference in its entirety.

### BACKGROUND

### 1. FIELD

The present disclosure relates to a centrifugal feeder that aligns and supplies objects by centrifugal force and to a tablet inspection device including the same.

### 2. DESCRIPTION OF RELATED ART

In general, tablets for medical purposes such as treatment are mass-produced through an automated process, and a process for inspecting defective products that occur during this production process is required.

For example, during the process of producing tablets in an automated process, tablets with external defects such as foreign substance attachment or contamination, cracks or partial damage due to contact or impact between tablets, deformation or poor printing are removed through a defective product inspection process.

The process of inspecting the appearance of capsules or tablets with an oval or circular cross-section is changing from visual inspection performed by workers to automatic inspection using a tablet inspection device. Various types of tablet inspection devices for this automatic inspection have been proposed.

In the case of automatically inspecting tablets using a tablet inspection device, a method is generally adopted in which a camera is used to capture an image of one side of the tablet during transport, and then the camera is used to capture an image of the other side of the tablet during tablet transport by reversing the tablet's posture, and then the images of the one side surface and the other side back are processed to inspect the presence or absence of defects.

As an example of such technology, Patent Document 1 discloses a centrifugal feeding device in which tablets are fed from a tablet input unit to a supply unit and a rotating plate, and the tablets supplied to the supply unit and the rotating plate are moved, in a row, to the upper portion of side surface of the supply unit by the rotating plate, while being in close contact with the side surface of the supply unit by centrifugal force and supplied to an external inspection device.

However, a guide cover is provided to guide tablets in a centrifugal feeding device of the related art, but the inherent surface frictional force is different depending on the manufacturing characteristics of the product, and in cases where the frictional force is small, there is no supply problem, but in cases where the frictional force is large, the alignment may become poor or the supply speed is significantly reduced due to the frictional force between the guide and the product. In addition, the tablet may be caught in the guide cover while being transported at a high speed and may be damaged, and when the tablet is damaged, not only the broken tablet but also the broken fragments may be attached to the surrounding tablets, which not only damages a large number of tablets, but in severe cases, the supply of tablets is blocked, so that the user should remove the tablets caught in the guide cover, which causes a problem in that the work efficiency is reduced.

Meanwhile, in the case of centrifugal feeding devices, tablets are supplied quickly and at uneven intervals, placing a load on subsequent inspection equipment, requiring a high level processor. Accordingly, a technology, for example, Patent Document 2, has been developed in which a groove is formed in a transport device without using a centrifugal feeding device, and tablets are moved by entering the groove. However, since the drum of the feeder should be replaced for each tablet type, the time/cost for replacing the drum may be significant, and it has the limitation of being unsuitable for inspecting various types of tablets.
(Patent Document 1) KR 10-1689281 B
(Patent Document 2) KR 10-2167067 B

### SUMMARY

An aspect of the present disclosure is to provide a centrifugal feeder capable of rapidly supplying tablets at a constant interval and/or at a constant posture without damage to tablets during a supply process, and a tablet inspection device including the same.

According to some embodiments, the following centrifugal feeder and a table inspection device are provided.

According to an aspect of the present disclosure, a centrifugal feeder includes an inner disk having tablets supplied thereto and rotating with a central axis thereof inclined relative to a vertical direction; an outer disk rotating around while surrounding the inner disk and including a tablet moving surface in which the tablets received from the inner disk are moved; and a pocket guide connected to the outer disk, covering a portion of a tablet moving surface and including a plurality of pockets into which the tablets are received.

The pocket guide may include a cover portion covering an outer surface of the outer disk and a pocket portion connected to the cover portion and extending to the tablet moving surface to form the plurality of pockets.

The pocket guide may be configured to be replaceable from the outer disk.

The pocket guide may include a protrusion protruding from one side connected to the cover portion toward a center of rotation of the outer disk and partitioning neighboring pockets, and a width of the protrusion may decrease in a circumferential direction as approaching the center of rotation.

A pitch from a vertex of the protrusion to a vertex of a neighboring protrusion may be smaller than twice a maximum width of the tablets supplied to the centrifugal feeder.

The protrusion may include a first curved surface having a first radius of curvature with respect to a neighboring protrusion.

The protrusion may include a plane extending obliquely from the first curved surface.

A vertex of the protrusion may be formed as a second curved surface having a second radius of curvature, and the first radius of curvature may be larger than the second radius of curvature.

The plane may be inclined at a first angle (Θ) with respect to a radial direction of the pocket guide, the first angle may be greater than 0° and less than or equal to 60°, and in detail, the first angle may be between 20° and 40°.

A protrusion height of the protrusion may be less than or equal to twice the first radius of curvature and greater than or equal to the first radius of curvature, and in detail, the protrusion height may be between 4/3 times and 1.5 times the first radius of curvature.

The cover portion may be formed by bending to cover a portion of a lower surface in addition to the outer surface of the outer disk.

The centrifugal feeder may further include a fixed frame surrounding the outer disk; and a guide connected to the fixed frame, positioned by a second angle from a top dead point of the inner disk in a direction opposite to a rotational direction of the outer disk when viewed from above, being a portion in which the inner disk and the outer disk come into contact in a radial direction, and including a guide surface protruding from a starting position of a height of the outer disk in a vertical direction to an area of the inner disk in the rotational direction.

At the starting position of the guide surface, the outer disk may be positioned higher than the inner disk by a first distance, and the first distance may be between 1/2 and 9/10 of a width of a target tablet.

The guide surface may have an angle formed with a horizontal plane and decreasing gradually in the rotational direction.

The guide may include a first portion having a protrusion height inwardly in a radial direction, increasing in the rotational direction, and a second portion having a protrusion height inwardly in the radial direction, decreasing in the rotational direction. The second portion may be positioned rearward of the first portion in the rotational direction.

The first portion may include a first vertical surface connected to the guide surface and extending in a vertical direction.

An end position of the guide surface in the rotational direction may be positioned in a position of the top dead point of the inner disk or spaced apart from the position of the top dead point of the inner disk in the direction opposite to the rotational direction.

The guide may be positioned 5 to 10° ahead of the top dead point of the inner disc.

The centrifugal feeder may further include a gate connected to the fixed frame and positioned rearward of the top dead point in the rotational direction, and the gate may include a first gate portion extending in a circumferential direction, outwards further than an inner surface of the outer disk in the radial direction, and a second gate portion connected to the first portion and protruding inwardly from the inner surface of the outer disk in the radial direction, along the rotational direction.

The first gate portion may be disposed above the tablet moving surface, and a distance from a lower end of the first gate portion to an upper surface of the outer disk may be shorter than a shortest distance among a height, a width, and a length of the tablets.

### BRIEF DESCRIPTION OF DRAWINGS

The above and other aspects, features, and advantages of the present disclosure will be more clearly understood from the following detailed description, taken in conjunction with the accompanying drawings, in which:
FIG. 1 is a schematic front view of a tablet inspection device according to an embodiment;
FIG. 2 is a schematic plan view of a tablet inspection device according to an embodiment;
FIG. 3 is a schematic perspective view of a centrifugal feeder included in a tablet inspection device according to an embodiment;
FIG. 4 is another schematic perspective view of a centrifugal feeder according to an embodiment;
FIG. 5 is a plan view of a pocket guide of a centrifugal feeder according to an embodiment;
FIG. 6 is a perspective view of a pocket guide of a centrifugal feeder according to an embodiment;
FIGS. 7 to 9 are partially enlarged views of pocket guides of a centrifugal feeder according to embodiments;
FIGS. 10 to 15 are modified examples of pocket guides of a centrifugal feeder according to an embodiment;
FIG. 16 is a plan view of a centrifugal feeder according to an embodiment;
FIG. 17 is a partial perspective view of a centrifugal feeder according to an embodiment;
FIG. 18 and FIG. 19 are schematic views of tablets used in the present disclosure;
FIG. 20 is a partial cross-sectional view taken along line A-A' of FIG. 17;
FIG. 21 is a partial cross-sectional view taken along line B-B' of FIG. 17;
FIG. 22 and FIG. 23 are partial perspective views of modified examples of the centrifugal feeder according to an embodiment;
FIG. 24 is a partial perspective view of a centrifugal feeder according to an embodiment;
FIG. 25 is a front view of an inner plate of a gate of FIG. 24;
FIG. 26 to FIG. 28 are partial cross-sectional views taken along lines C-C' to E-E' of FIG. 13;
FIG. 29 is a schematic perspective view of a vibrating screw supply unit according to an embodiment;
FIG. 30 is an exploded perspective view of a vibrating screw supply unit according to an embodiment; and
FIG. 31 is a cross-sectional view taken along line F-F' of FIG. 30.

### DETAILED DESCRIPTION

Hereinafter, example embodiments will be described with reference to the attached drawings. However, the embodiments may be modified in various other forms, and the scope of the present disclosure is not limited to the embodiments described below.

In addition, the embodiments are provided to more completely explain the present disclosure to those with average knowledge in the relevant technical field.

In the drawings, the shapes and sizes of elements may be exaggerated for clearer explanation.

In describing the embodiments, if it is determined that a detailed description of known technologies related to the present disclosure may unnecessarily obscure the gist of the present disclosure, the detailed description thereof will be omitted. In addition, the terms described below are terms defined in consideration of their functions in the present disclosure, and may vary depending on the intention or custom of the user or operator. Therefore, the definitions should be made based on the contents throughout this specification. The terms used in the detailed description are only for describing the embodiments and should never be limited. Unless clearly used otherwise, expressions in the singular form include plural meanings.

In this description, expressions such as "including" or "provided" are intended to indicate certain characteristics, numbers, steps, operations, elements, parts or combinations thereof, and should not be construed to exclude the presence or possibility of one or more other characteristics, numbers, steps, operations, elements, parts or combinations thereof other than those described.

Unless otherwise specifically provided in the specification of the present disclosure, the % unit means weight %.

In this specification, terms such as "on", "above", "upper portion", "upper surface", "top", "below", "lower portion", "lower surface", "side", and the like are based on the drawings, and may actually vary depending on the direction in which the elements or components are disposed.

In addition, throughout the specification, when a part is said to be "connected" to another part, this includes not only cases where it is "directly connected", but also cases where it is "indirectly connected" with another element therebetween.

Below, the present disclosure will be described in detail through each embodiment or example of the present disclosure. It should be noted that each embodiment or example described in this specification is not limited to a single embodiment or example, but may be combined with other embodiments or examples. Therefore, the citation of a claim in the scope of the patent claims is only an example of an embodiment, and the technical idea of the present disclosure should not be interpreted only as in combination with the cited claim, and the combination with various claims is also included in the scope of the technical idea of the present disclosure.

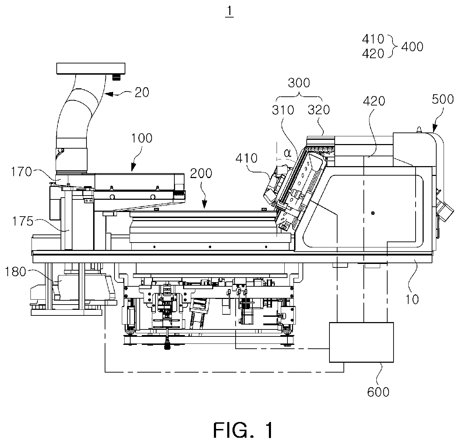

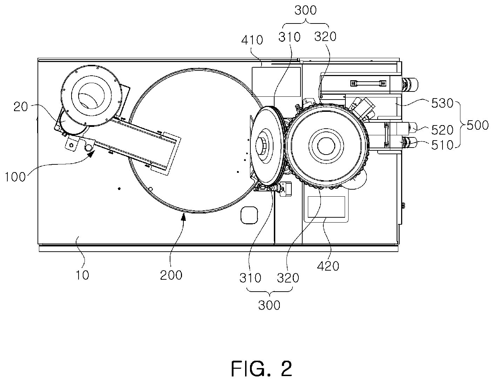

FIG. 1 and FIG. 2 illustrate a schematic front view and a plan view of a tablet inspection device according to an embodiment.

As illustrated in FIGS. 1 and 2, a tablet inspection device 1 includes a vibrating screw supply unit 100 connected to a hopper provided inside or outside the device, receiving tablets from a hopper connection pipe 20 that supplies tablets into the device, and supplying the tablets at a set speed; a centrifugal feeder 200 that aligns tablets delivered from the vibrating screw supply unit 100 through rotating inner disk 220 and outer disk 230 and supplies the same to a moving portion 300; a moving portion 300 including a first moving portion 310 absorbing and moving tablets that are aligned and supplied from the centrifugal feeder 200 and a second moving portion 320 reversing and receiving tablets from the first moving portion 310; an inspection unit 400 inspecting tablets while they are being moved through the moving portion 300, and including a first inspection unit 410 inspecting tablets moved in the first moving portion 310 and a second inspection unit 420 inspecting tablets moved in the second moving portion; a control unit 600 connected to the vibrating screw supply unit 100, the centrifugal feeder 200, the moving portion 300 and the inspection unit 400, and determining whether there is a defect or whether the printing is good or bad based on the result of the inspection unit 400; and a classification unit 500 classifying and discharging the tablets based on the result determined by the control unit 600. Although not illustrated, the tablet inspection device 1 may include a tablet printing unit printing on the tablet using a laser while the tablet is being moved by the moving portion 300, and may also include a pre-inspection unit determining the position/posture of the tablets before printing in the tablet printing unit.

In the tablet inspection device 1, the tablet may move in the upper portion centered on a device frame 10, and various devices, such as a vacuum forming portion, a driving motor, and a control unit, may be disposed in the lower portion centered on the device frame 10.

The tablet may be broken by impact, and when broken in this manner, the broken powder may attach to other tablets, causing different dosages or poor printing or inspection. In addition, when the tablet is supplied through the centrifugal feeder 200, the posture and position may change due to centrifugal force, and in this case, it may be difficult for the inspection unit 400, the printing unit, or the control unit 600 disposed subsequently to inspect/print/determine the tablet. It is also possible to capture an image, analyze the image, and print or inspect the image at the correct location, but if the image goes through a complicated process, problems with accuracy may occur, determination may take time, or system requirements may increase.

Therefore, in addition to the method of supplementing with software, there is a demand for a device that controls the posture and position of tablets supplied by the device and prevents tablets from being broken, and the present disclosure may satisfy this demand.

The vibrating screw supply unit 100 supplies tablets supplied from the hopper to the centrifugal feeder 200 through vibration, and includes a screw together with a vibrating structure to not only transport the tablets, but also remove fragments and dust generated by tablet breakage without supplying the same to the centrifugal feeder 200.

The tablet is a solidified powder, and has the characteristic of being weak to impact regardless of whether it is coated. During the process of pouring the tablet into the hopper or during the process of being delivered to the vibrating screw supply unit 100 through a hopper supply pipe 20, the tablet may be broken by collision with the device or by collisions between tablets. The vibrating screw supply unit 100 removes such broken tablets and dust before supplying the tablets to the centrifugal feeder 200 and supplies a set amount to the centrifugal feeder 200. The vibrating screw supply unit 100 will be described again later with reference to the drawings.

The centrifugal feeder 200 rotates the inner disk 220 to transfer the tablets supplied from the vibrating screw supply unit 100 to the outer disk 230 through centrifugal force of the tablets, and then supplies the tablets to the moving portion 300 while the outer disk 230 rotates. In the present disclosure, the centrifugal feeder 200 includes at least one of a pocket guide 240, a guide 260, and a gate 270 in addition to the inner disk 220 and the outer disk 230, thereby supplying tablets supplied from the centrifugal feeder 200 at a set position and/or posture, and at the same time preventing damage to tablets that may occur during the process of rotating or aligning in the centrifugal feeder 200, thereby providing a centrifugal feeder 200 that is easy to manage and improves work efficiency. The centrifugal feeder 200 will also be described later with reference to the drawings.

The moving portion 300 has a rotating disk-shaped structure, and the inside thereof is connected to a vacuum forming portion, so that the groove formed on the attachment surface corresponding to the outer surface of the disk is in communication with the vacuum forming portion, and the disk rotates. The moving portion 300 has a structure that rotates the disk while suctioning tablets close to the attachment surface.

In the tablet inspection device 1 according to an embodiment of the present disclosure, the centrifugal feeder 200 is disposed on a plane, and is configured to deliver the tablets to the moving portion 300 in an inclined state with an inclination angle (α) toward the outside from the centrifugal feeder 200 so that the tablets may be easily aligned. Accordingly, the first moving portion 310 of the moving portion 300 is configured in a disk-shaped shape and has an attachment surface parallel to the rotation axis, so that the tablets are delivered in an inclined state, and the tablets are disposed to be inclined by the inclination angle (α) with respect to the vertical direction.

The second moving portion 320 is configured in a disk-shaped shape, has a vertical rotation axis, and the attachment surface thereof is configured to be inclined by the inclination angle (α) with respect to the vertical direction, so that the tablets are transferred from the first moving portion 310 in an inclined state with respect to the vertical direction. The tablets are reversed as they pass from the first moving portion 310 to the second moving portion 320.

In the present disclosure, since the first moving portion 310 is inclined with respect to the vertical direction and the second moving portion 320 is disposed horizontally, the height of the tablet inspection device 1 may not increase, but a space in which the inspection unit 400 or the printing part is disposed may be secured. In the case of the tablet inspection device 1, continuous management by the user is required, but if the height of the tablet inspection device 1 increases, management becomes difficult and the time required for management increases, but through the arrangement of the first and second moving portions 310 and 320, management becomes convenient while securing space.

The inspection unit 400 includes a plurality of cameras, and includes a first inspection unit 410 inspecting tablets moved by the first moving portion 310 and a second inspection unit 420 inspecting tablets moved by the second moving portion 320. The inspection unit 400 may further include a pre-first inspection unit inspecting the tablet upstream of the printing unit in the first moving portion 310 when the tablet inspection device 1 includes a printing unit, and the pre-first inspection unit photographs the tablet before printing and provides the photograph to the control unit 600, thereby enabling correction of printing in the printing unit. Similarly, the inspection unit 400 may further include a pre-second inspection unit inspecting the tablet upstream of the printing unit in the second moving portion 320. The inspection unit 400 may typically be configured with a combination of a 3D camera and a 2D camera.

The classification unit 500 includes first to third classification units 510, 520 and 530 that determine whether the tablet is good or bad through the image captured by the inspection unit 400 and collect good, defective, and unclassified tablets. The classification unit 500 may include air nozzles corresponding to the first to third classification units 510, 520 and 530, and the air nozzle blows air onto the tablet attached to the attachment surface of the second moving portion 320 to cause the tablet to fall into one of the first to third classification units 510, 520 and 530.

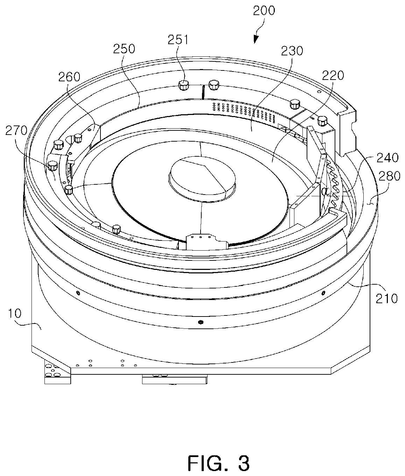

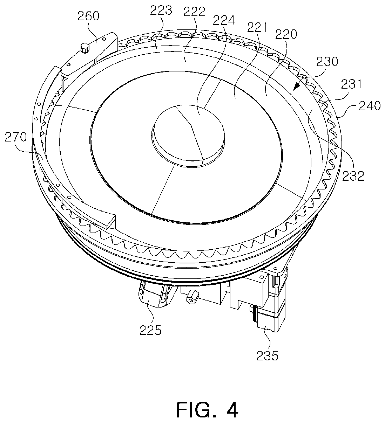

FIG. 3 is a schematic perspective view of a centrifugal feeder included in a tablet inspection device according to an embodiment, and FIG. 4 is another schematic perspective view of a centrifugal feeder according to an embodiment.

As illustrated in FIGS. 3 and 4, the centrifugal feeder 200 includes a fixed frame 210 installed on the device frame 10, surrounding the inner and outer disks 220 and 230, and connected to the device frame 10; an inner disk 220 disposed inside the fixed frame 210 and rotating around a rotation axis inclined with respect to the vertical direction; an outer disk 230 surrounding the inner disk 220 and rotating around a rotation axis parallel to the vertical direction, as a center (CoD); a pocket guide 240 mounted on the outer periphery of the outer disk 230; a cover portion 250 connected to the fixed frame 210 and covering the upper surface of the rotating outer disk 230; a guide 260 including a guide surface 262 protruding from the outer disk 230 toward the inner disk 220, and connected to the cover portion 250 or the fixed frame 210; and a gate 270 positioned rearward in the rotational direction of the outer disk relative to the guide 260 and dropping tablets other than those in a predetermined posture on the outer disk 230 back to the inner disk 220. In addition, the centrifugal feeder 200 may also include an air nozzle 280 for spraying air to the tablet protruding from the outer disk 230 behind the gate 270.

The fixed frame 210 is connected to the device frame 10 and forms the outer shape of the centrifugal feeder 200. The fixed frame 210 has a cylindrical shape, and rotatably supports the inner frame 220 and the outer frame 230, and other non-rotatable configurations may be fixedly mounted thereon.

The inner disk 220 has a rotation axis inclined with respect to the vertical direction, is connected to an inner disk driving unit 225, and is coupled to the inner disk driving unit 225 through a coupling portion 224. The inner disk 220 may be configured to have one upper surface, but may be configured to have multiple surfaces, and in this embodiment, has first to third surfaces 221, 222 and 223. The third surface 223 may be inclined to correspond to a tablet moving surface 231 of the outer disk 230 from the top dead point (TDP) of the inner disk 220. In detail, the first to third surfaces 221 to 223 are configured such that the inclination angles are sequentially gentler with respect to the rotation axis of the inner disk 220.

The outer disk 230 has a rotation axis parallel to the vertical direction and is connected to an outer disk drive unit 235. As illustrated in FIG. 4, the outer disk drive unit 235 has a structure that rotates the outer disk 230 through a gear below the outer disk 230, but is not limited thereto. The outer disk 230 has a ring-shaped upper surface surrounding the inner disk 220, and the upper surface includes the tablet moving surface 231, inclined so that the height increases inwardly with respect to the rotation axis, for example, the height decreases toward the outside in the radial direction. The tablet moving surface 231 is configured to be inclined by an inclination angle (α) with respect to the horizontal plane. The tablet moving surface 231 includes a vertical surface 232 extending vertically on the inner side of the tablet moving surface 231. The vertical surface 232 and the inner disk 220 cooperate to provide a space where the tablets supplied to the centrifugal feeder 200 stay.

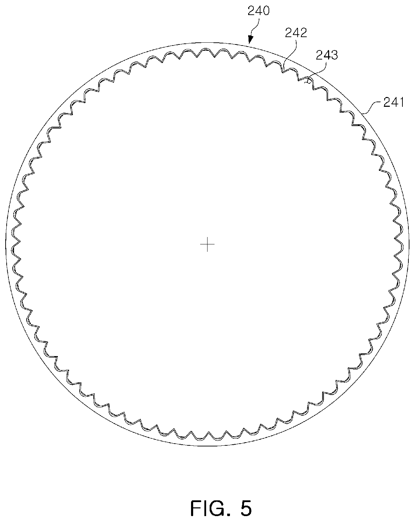

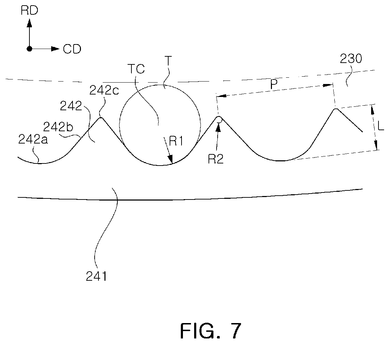

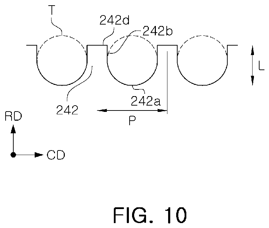

The pocket guide 240 will be described with reference to FIGS. 5 to 15. FIGS. 5 to 6 illustrate plan views and perspective views of a pocket guide of a centrifugal feeder according to an embodiment, FIGS. 7 to 9 illustrate partially enlarged views of a pocket guide of a centrifugal feeder according to an embodiment, and FIGS. 10 to 15 illustrate modified examples of a pocket guide of a centrifugal feeder according to an embodiment.

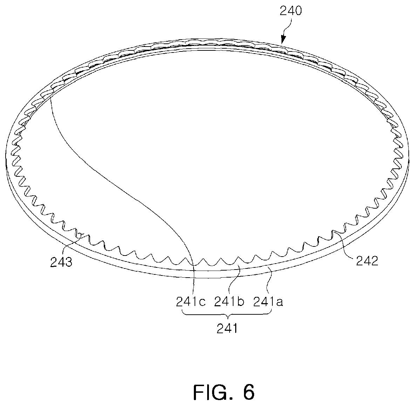

As illustrated in FIGS. 5 and 6, the pocket guide 240 includes a pocket cover portion 241 that covers a portion of the tablet moving surface 231 of the outer disk 230 and an outer periphery of the outer disk 230, and the pocket guide 240 is configured to be coupled to the outer disk 230 and rotate together. The pocket guide 240 includes protrusions 242 protruding from the pocket cover portion 241 to form a plurality of pockets 243 receiving tablets in certain positions when the tablets pass from the inner disk 220 to the tablet moving surface 231 of the outer disk 230. The pocket guide 240 is configured to have a predetermined thickness, and it may be preferable that the thickness thereof be at least 1/2 of the thickness of the tablet so that the tablets do not fall out to the outside beyond the pocket guide 240.

The pockets 243 are formed in a plurality at a certain interval along the circumferential direction. For example, the protrusions 242 forming the pockets 243 protrude from the pocket cover portion 241 at a certain interval. In the present disclosure, by forming the pockets 243, the tablets may be received only inside the pockets 243, thereby limiting the position at which the tablets are placed on the tablet moving surface 231 and also limiting the posture of the tablets. Therefore, the load on the inspection unit 400 may be reduced by supplying the tablets in a set position and posture to the moving portion 300 receiving the tablets from the outer disk 230.

The pocket guide 240 rotates together with the outer disk 230, but is configured to be replaceable from the outer disk 230. For example, the inner surface of the pocket guide 240 is fitted into the outer disk 230, to rotate together therewith, and when a change is required, the pocket guide 240 is separated from the outer disk 230 and another pocket guide 240 is fitted and used.

The pocket cover portion 241 covers the outer surface of the outer disk 230, and includes a first portion 241a that allows the pocket guide 240 to be fitted into the outer disk 230, and a second portion 241b that is bent and extended from the first portion 241a and is disposed on the tablet moving surface 231 and connected to the protrusions 243. In addition, the pocket guide 240 may include a third portion 241c (see FIG. 6), bent from the lower portion of the first portion 241a and bent to the lower surface of the outer disk 230.

As illustrated in FIG. 7, the protrusion 242 forms a pocket 243 together with the neighboring protrusion 242. The protrusion 242 is configured such that a width thereof in the circumferential direction decreases toward the center of rotation to facilitate the introduction of the tablet (T) into the pocket 243. The protrusion 242 includes a first curved surface 242a having a first radius of curvature R1, and a plane 242b extending in a straight line from the first curved surface 242a toward the vertex of the protrusion to be inclined at a first angle (Θ) with respect to the radial direction (RD) of the outer disk 230. It may be preferable that the first radius of curvature R1 has a radius corresponding to the radius of the tablet (T), but the pocket guide 240 may be used corresponding to a tablet (T) having a certain range of radii. The first radius of curvature R1 may have a radius of about an intermediate value in the radius range of the tablet (T) to be used.

By forming the protrusion 242 with the first curved surface 242a and the plane 242b in this manner, the center (TC) of the tablet (T) may be positioned only at a distance corresponding to the radius of the tablet (T) from the inner side surface of the pocket cover portion 241. For example, the pocket guide 240 rotates together with the outer disk 230, and the tablet moving surface 231 of the outer disk 230 is configured to be inclined outwardly, and thus, the tablet that has come up on the tablet moving surface 231 is pushed outward within the pocket 243 by the plane 242b and the first curved surface 242a. Therefore, the position of the tablet (T) may always be positioned at a constant position.

However, both the first curved surface 242a and the plane 242b are not essential, and the protrusion 242 may be formed only with the first curved surface 242a, or the protrusion 242 may be formed only with the plane 242b without the first curved surface 242a. For example, the protrusion 242 may have various structures as long as it may form a pocket 243.

To form a pocket 243 that accommodates a polygonal tablet, the protrusion 242 may be protruded along the first curved surface 242a corresponding to a circle or an ellipse passing through the vertex of the polygon, and may also protrude with a plane 242b corresponding to a side of the polygon instead of a curved surface.

The protrusion 242 has a pitch (P) as the distance between the vertices, and the pitch (P) may be constant, and may be preferably 4 times the radius of the tablet (T), for example, twice or less the diameter of the tablet (T), and should be at least 2 times the radius of the tablet (T), for example, exceed the diameter of the tablet (T), so that the tablet (T) may enter. As mentioned above, when the pocket guide 240 is used for a certain range of the tablet (T) radius, the pitch (P) may be determined based on a maximum radius.

In addition, the protrusion height (L) of the protrusion 242 may be, in detail, equal to or greater than the first radius of curvature R1 of the first curved surface 242a corresponding to the radius of the tablet (T), and 2 times or less of the first radius of curvature R1, and in more detail, may be equal to or greater than 4/3 and 1.5 times or less of the first radius of curvature R1. The protrusion height (L) of the protrusion 242 is related to the first angle (θ) and the pitch (P). If the protrusion height (L) is relatively low, the tablet (T) may move to the neighboring pocket 243 during the rotation of the outer disk 230, and in a case in which the tablet (T) is absorbed by the moving portion 300 during the movement, the tablet (T) may not be supplied to the designated position despite the presence of the pocket 243. On the other hand, if the protrusion height exceeds twice the first radius of curvature R1, the pitch (P) increases, so that the number of tablets (T) supplied by the pocket guide 240 decreases at the same rotation speed, and as the protrusion height (L) is high, it may become difficult for the tablet to enter the pocket 243, and thus, the number of supplied tablets (T) decreases, which may lower the overall supply efficiency.

As illustrated in FIG. 7, when a circular tablet is received in the pocket 243 formed by the protrusion 242, the position of the center (TC) of the tablet (T) is always located at a constant distance from the center of rotation of the outer disk 230, and therefore, the position of the tablet received from the moving portion 300 may also be constant.

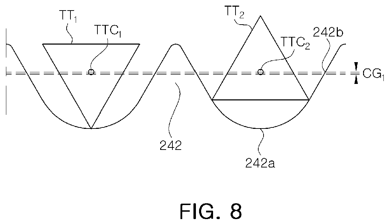

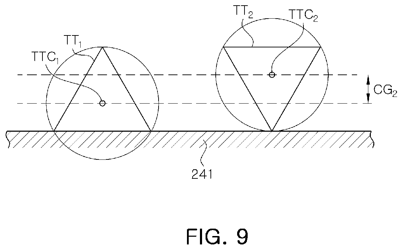

FIG. 8 illustrates the center position when a triangular tablet (TT) is located in the pocket 243, and FIG. 9 illustrates the center position of a triangular tablet (TT) when there is no pocket 243 formed by a protrusion 242.

In the case of a circular tablet, even if there is no protrusion 242, the center position may be constant in the transfer line due to the pocket cover portion 241. However, as illustrated in FIG. 9, when the triangular tablet (TT) is aligned by the pocket cover portion 241 in the absence of the protrusion 242, the difference CG2 between the centers TTC1 and TTC2 in the case TT1 in which the edge of the tablet touches the pocket cover portion 241 and in the case TT2 in which the vertex touches the pocket cover portion 241 is formed significantly, and the difference CG2 is approximately 1/4 of the radius of the circle passing through the vertex of the triangular tablet. However, as illustrated in FIG. 8, when the pocket 243 is formed by the protrusion 242, the vertex of the triangular tablet (TT) may be located further outside than the sides. Therefore, in the case TT2 in which the corner touches the pocket cover portion 241, the difference CG1 between the centers TTC1 and TTC2 may be reduced compared to the difference CG2 of FIG. 9, and the difference CG1 is approximately 1/60 of the radius of the circle passing through the vertex of the triangular tablet. Therefore, by forming a pocket 243 through the protrusion 242, the tablet (TT) may be provided to the moving portion 300 in a state where the center deviation of the tablet is relatively small in the centrifugal feeder 200.

Meanwhile, the vertex of the protrusion 242 may be formed sharply by meeting the plane 242b, but may also be formed as a second curved surface having a second radius of curvature, or as a top surface 242d (see FIG. 10) parallel to the circumference direction. In this case, it may be preferable that the second radius of curvature is smaller than the first radius of curvature R1.

FIGS. 10 to 15 illustrate variations of a pocket guide 240 according to an embodiment.

As illustrated in FIG. 10, a protrusion 242 is formed to form a pocket 243 corresponding to a tablet so that a tablet (T) may be inserted. The protrusion 242 includes a first curved surface 242a having a first radius of curvature R1 corresponding to the radius of the tablet (T), a plane 242b extending parallel to the radial direction (RD), and a top surface 242d parallel to the circumferential direction (CD) between the planes 242b. In this variation, insertion of the tablet (T) into the pocket 243 is difficult, but the tablet (T) may always be located at the center of the pocket 243.

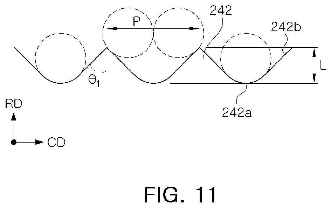

As illustrated in FIG. 11, in the present disclosure, the pocket guide 240 may have a protrusion 242 in which the plane 242b of the protrusion 242 is inclined with respect to the radial direction (RD), the protrusion height (L) of the protrusion 242 is smaller than the diameter of the tablet (T), and the pitch (P) is 4 times or less the radius of the tablet (T). In this case, two tablets (T) cannot enter one pocket 243 and are caught on the protrusion 242, and the tablets caught on the protrusion 242 in this way are protruded from the tablet moving surface 231 toward the inner disk 220 and fall. Therefore, by controlling the height of the protrusion 242, multiple tablets may be prevented from entering one pocket 243 and being supplied to the moving portion 300.

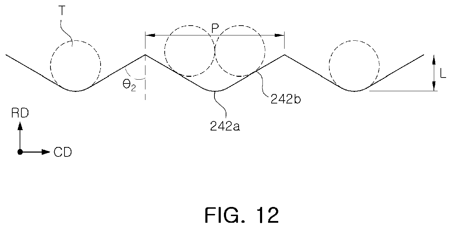

On the other hand, as illustrated in FIG. 12, the pocket guide 240 may have a protrusion 242 whose plane 242b of the protrusion 242 is inclined with respect to the radial direction (RD), whose protrusion height (L) is smaller than the diameter of the tablet (T), and whose pitch (P) is more than four times the radius of the tablet (T). In this case, two tablets (T) are caught on the plane 242b of one pocket 243, and the tablets caught on the plane 242b of the protrusion 242 protrude slightly or do not protrude from the tablet moving surface 231 toward the inner disk 220, so that the tablets may be supplied to the moving portion 300 without falling onto the inner disk 220.

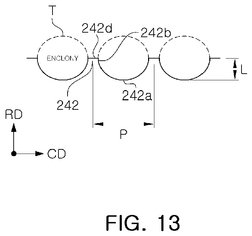

In the case in which the tablet is oval, it is also possible for the first curved surface 242a to be formed corresponding to the oval tablet (T), as illustrated in FIG. 13. In this case, the height (L) of the protrusion 242 may be from 1/2 or more of the length of the short axis of the oval tablet (T) to the length or less of the short axis.

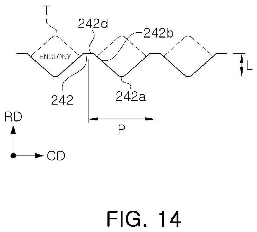

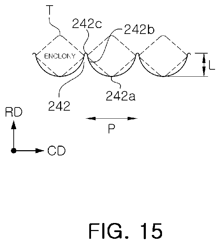

In FIGS. 14 and 15, the shapes of the protrusion 242 in the case in which the tablet is a quadrangle, for example, a diamond-shaped tablet (T) are illustrated. As in FIG. 14, in the case of a diamond-shaped tablet (T), the protrusion 242 may be formed to have a shape formed of a first curved surface 242a, a plane 242b, and a second curved surface 242c to correspond to the shape of the tablet (T), but as in FIG. 15, may also be formed of a first curved surface 242a and a second curved surface 242c to correspond to an oval surrounding the vertex of the diamond-shaped tablet (T), or formed of a first curved surface 242a and a top surface 242d (see FIG. 10). At this time, the height of the protrusion 242 may be determined in proportion to the short-axis length of the oval, similar to the oval tablet of the above-mentioned FIG. 13.

Table 1 records the diameter (ø) of the tablet (T) to be inspected, the height (L) of the protrusion 242 of the pocket guide 240, and the supply status at the pitch (P). The pocket cover portion 241 is formed such that the distance from the inner side of the tablet moving surface 231 of the outer disk 230 to the lower surface of the protrusion 242 of the pocket guide 240 is larger than the diameter (ø) of the tablet. Good means that the tablet (T) is inserted into each pocket and is transferred to the moving portion 300 with the center of the tablet (T) constant, and insufficient supply indicates that the tablet (T) is not inserted into some pockets, and poor position means that the tablet (T) is transferred to the moving portion 300 with the center not constant.

**[Table 1]**

| | Diamet er (mm) | First radius of curvature (R1, mm) | Height( L, mm) | First Angle ( ° ) | Supply Status |
|---|---|---|---|---|---|
| Compar ative Exampl e 1 | 10 | 5 | 3 | 80 | Poor Positio n |
| Exampl e 1 | 10 | 5 | 5 | 60 | Good |
| Exampl e 2 | 10 | 5 | 8 | 40 | Good |
| Exampl e 3 | 10 | 5 | 10 | 20 | Good |
| Exampl e 4 | 10 | 5 | 12 | 0 | Insuffi cient Supply |
| Compar ative Exampl e 2 | 8 | 4 | 5 | 70 | Poor Positio n |
| Exampl e 5 | 8 | 4 | 6 | 40 | Good |

Comparative Example 1 and Examples 1 to 4 are the results of tests conducted while changing the height (L) and pitch (P) for tablets of the same diameter, and Comparative Example 2 and Example 5 are the results of tests conducted on tablets (T) of different diameters from Examples 1 to 4. In Comparative Examples 1 and 2 and Examples 1 to 3 and 5, a plane 242b was configured to be inclined at a first angle (Θ) with respect to the radial direction (RD) without a top surface 242d, and as the first angle (Θ) was formed larger, the pitch (P) increased. In the cases of Comparative Examples 1 and 2, there were cases where multiple tablets (T) entered one pocket 243, and in the case of Example 4, as illustrated in FIG. 10, a plane 242b was configured parallel to the radial direction (RD), and a protrusion 242 having a top surface 242d was applied. In this case, the supply shortage occurred because the tablet (T) was not inserted into some pockets 243, but the position thereof was not bad.

Considering the test results, it was good in the supply state when the plane 242b was inclined, the first angle (θ) was 0° to 60°, and the protrusion height (L) was 2 to 4 times the first radius of curvature R1, and in more detail, when the first angle (θ) was 20° to 40°, and the protrusion height (L) was 4/3 to 1.5 times the first radius of curvature R1, it was good in terms of the supply state.

Returning to FIG. 3, the cover portion 250 is fixed to the fixed frame 210 with a bolt 251 and covers a portion of the tablet moving portion 231, on the outer disk 230. In detail, the cover portion 250 covers the outer disk 230 except for an opening portion 280 through which the guide 260, the gate 270, and the moving portion 300 adsorb the tablets on the tablet moving portion 231 of the outer disk 230.

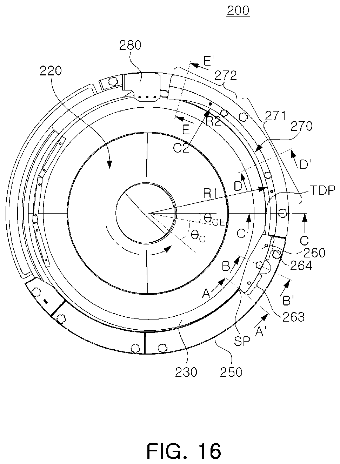

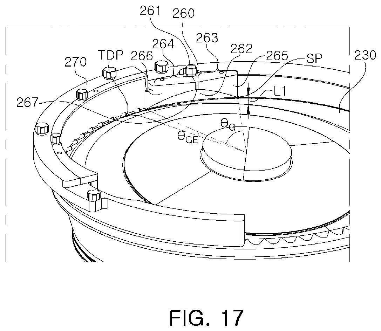

FIG. 16 is a plan view of a centrifugal feeder 200 according to an embodiment, and FIG. 17 is a partial perspective view centered on the guide 260 of the centrifugal feeder 200.

As illustrated in FIGS. 16 and 17, a guide 260 connected to a fixed frame 210 is disposed upstream of the top dead point (TDP) of the inner disk 220 in the rotational direction (RoD), and a gate 270 is disposed from the guide 260, to pass through the top dead point (TDP) and cover a certain area.

When the center of rotation (CoD) of the outer disk 230 is centered, the angle (θG) at which the guide 260 covers the upper surface of the outer disk 230 and the angle (θGE) between the guide 260 and the top dead point (TDP) are added, and the added angle is referred to as a second angle. The guide 260 starts at a position in a direction opposite to the direction of rotation by the second angle from the top dead point (TDP). The guide 260 is fixed to the cover portion 250 or the fixed frame 210 with a bolt 261 in the same manner as the cover portion 250.

The guide 260 includes a guide surface 262 that protrudes from the starting position (SP) that affects the tablet (T). The starting position (SP) is a position in a direction opposite to the direction of rotation by a second angle from the top dead point (TDP) in the rotational direction, is the height of the tablet moving surface 231 of the outer disk 230 in the vertical direction, and is the boundary between the inner disk 220 and the outer disk 230 in the radial direction, and the guide surface 262 is formed as a curved surface that protrudes toward the inner disk 220 from the starting position (SP) in the rotational direction.

Since the inner disk 220 and the outer disk 230 have the same height at the top dead point (TDP), the outer disk 230 is higher than the inner disk by a first distance L1 at the starting position (SP) of the guide surface 262. The first distance L1 may be smaller than the width (W) of the tablet (T), but may be preferably equal to or greater than 1/2 of the width (W) of the tablet (T). If it is less than 1/2, the lower portion of the tablet (T) comes into contact with the guide surface 262, so that the tablet (T) does not lie down inwardly of the inner disk 220, but instead falls over to the opposite side, and thus, the subsequent guide surface 262 may cause the tablet (T) to fall in the opposite direction to the laying direction, thereby causing the tablet (T) to be damaged by impact. In addition, it may be preferable that the first distance L1 is the width (W) or less of the tablet (T), but if it is greater than the width (W) of the tablet (T), the guide surface 262 rotates and acts in a manner that presses the top of the tablet (T), but in the case in which multiple tablets (T) move together, the tablets (T) are damaged by the pressing force. To stably lay down the tablet (T) through the guide surface 262, it may be preferable that the first distance L1 is 9/10 or less of the width (W) of the tablet (T).

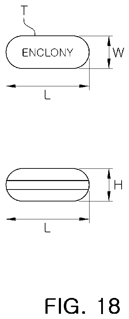

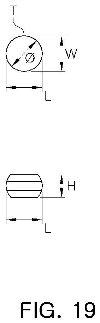

FIG. 18 and FIG. 19 illustrate a rectangular tablet (T) and a circular tablet (T). The width (W) of the tablet (T) means the short side when viewed from the printing surface direction in the case of a rectangular tablet, and means the diameter (φ) in the case of a circular tablet. In the present disclosure, the centrifugal feeder 200 should deliver the tablet to the moving portion 300 with the printing surface or the opposite surface of the printing surface facing upward so that printing is possible on the printing surface while passing through the moving portion 300. Therefore, in the case where the printing surface is not facing upward but standing, it is necessary to lay down the tablet (T), and the guide 260 plays a role in increasing the probability that the standing tablet (T) will lie down through the guide surface 262.

Returning to FIG. 17, the guide 260 includes first and second vertical surfaces 265 and 266 connected to the guide surface 262 and extending in the vertical direction, and the first vertical surface 265 guides the stacked tablets (T) to be in a single layer. In the upper space of the inner disk 220, the tablets (T) may be rotated in a state where they are stacked in multiple layers rather than in a single layer. Since the tablets (T) may be damaged by friction between the tablets during the process of falling after being transferred to the outer disk 230 in a state of being stacked in multiple layers, the first vertical surface 265 of the guide 260 also plays a role in organizing the tablets (T) moving in a state of being stacked in multiple layers so that only one layer remains.

The guide 260 includes a guide surface 262 that forms a curve surface in the rotational direction by starting from the starting position (SP), a first vertical surface 265 that extends vertically from the guide surface 262 in the first portion 263, the front part of the guide, a second vertical surface 266 that extends vertically from the guide surface 262 in the second portion 264, the rear part of the guide, and a bolt 261 that fixes the guide.

The guide 260 protrudes in the rotational direction toward the inner disk 220 in the first portion 263, and the protrusion length thereof decreases again in the second portion 264, and the guide 260 no longer protrudes toward the inner disk 220 at the end of the guide 260. If necessary, the guide 260 may include a maintenance surface 267 that maintains the protrusion length between the first vertical surface 265 and the second vertical surface 266.

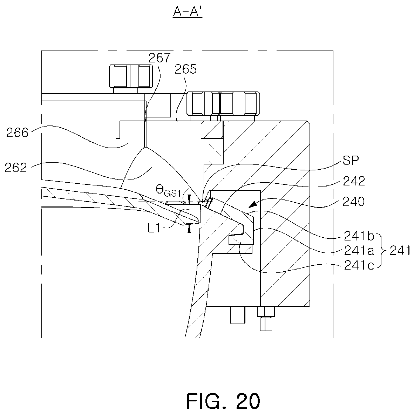

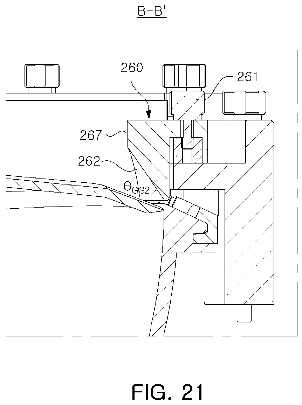

FIGS. 20 and 21 illustrate cross-sectional views taken along lines A-A' and B-B' of FIG. 17.

As illustrated in FIGS. 20 and 21, the guide surface 262 is curved, but is inclined at angles θGS1 and θGS2 with respect to the horizontal plane when viewed in cross-section. The curve of the guide surface 262 gradually decreases at the inclination angles θGS1 and θGS2 along the rotation direction (RoD). For example, the inclination angle θGS1 formed between the horizontal plane and the guide surface 262 at the starting position (SP) is greater than the inclination angle θGS2 formed between the horizontal plane and the guide surface 262 at the intermediate position (θGS1>θGS2).

As the inclination angle formed between the guide surface 262 and the horizontal plane along the rotation direction decreases, the guide surface 262 causes the standing tablet (T) to lie down in the rotation direction.

The guide surface 262 initially guides the tablet (T) to fall from the middle of the standing tablet (T) to the inside of the inner disk 220, and gradually lays down the tablet (T) through the inclination of the guide surface 262 while following the direction of rotation. Therefore, the tablet (T) passes through the guide 260 in any state without being damaged and is stably laid down by the guide surface 262 and emerges therefrom.

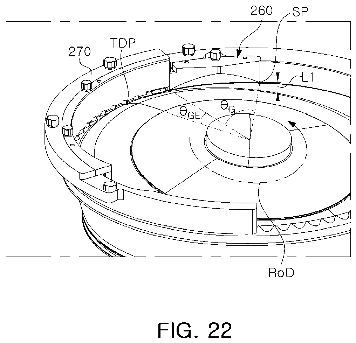

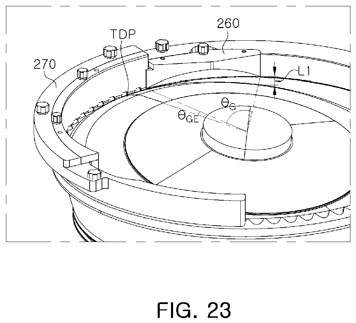

The starting position of the guide 260 may be important depending on the size of the tablet (T), and since the size of the tablet (T) to be inspected may be changed, the starting position (SP) of the guide 260 may also be changed, and the guide 260 is replaceably mounted on the centrifugal feeder 200. The guide 260 is configured to have a different length in the circumferential direction of the outer disk 230 so that the starting position (SP) may be changed depending on the size of the tablet, and FIGS. 22 and 23 are perspective views illustrating a guide 260 that has been replaced due to a change in the size of the tablet (T) .

As illustrated in FIG. 22, when the size of the tablet (T) increases, the circumferential length of the gate 270 is lengthened, so that the angle (θGE) formed by the end position of the guide 260 at the top dead point (TDP) increases. Accordingly, the starting position (SP) of the guide 260 located upstream of the gate 270 in the rotational direction (RoD) may be moved without changing the length of the guide 260.

Alternatively, as illustrated in FIG. 23, the starting position (SP) of the guide 260 may be moved by increasing the length of the guide 260 without changing the gate 270 and increasing the angle (θG) occupied by the guide 260 in the outer disk 230.

Even when the tablet (T) is changed, the first distance L1 at the starting position (SP) of the guide 260 is maintained to be between 1/2 and 9/10 of the width (W) of the tablet (T), so that the tablet (T) may be guided to lie down without damage while passing through the guide 260.

Meanwhile, it may be preferable that the guide 260 ends 5 to 10° before the top dead point (TDP), so that the tablet (T) is laid down by the guide 260 and passes through the top dead point (TDP) to move from the inner disk 220 to the outer disk 230. If the end point is less than 5°, the posture of the tablet (T) having passed through the guide 260 is not stable, and thus, there is a high possibility that the tablet's posture will change during the movement, and if it exceeds 10°, the tablet that has been laid down may have its position disturbed again by another tablet.

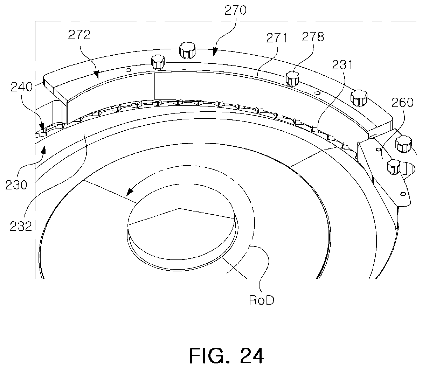

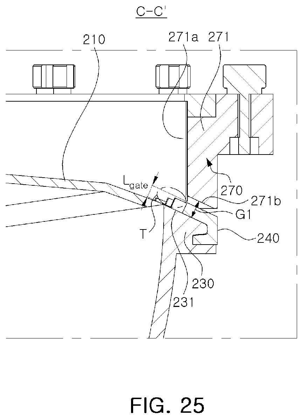

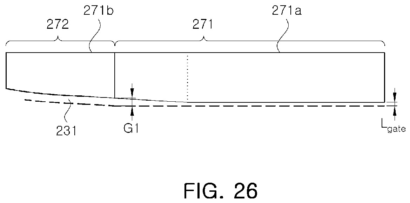

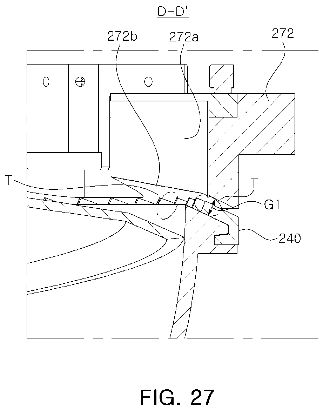

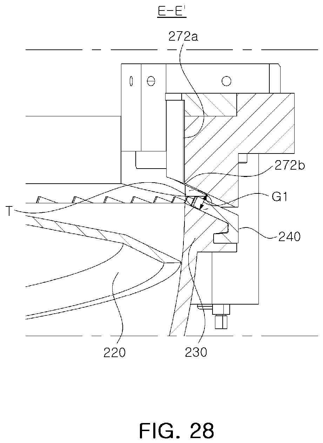

FIG. 24 is a partial perspective view of a centrifugal feeder according to an embodiment, and FIG. 25 is a front view of a gate 270. FIG. 24 is a partial perspective view centered on the gate 270. FIGS. 26 to 28 illustrate cross-sectional views taken along lines C-C', D-D', and E-E' in FIG. 16.

The gate 270 is connected to the fixed frame 210 and is positioned rearward of the guide 260 in the rotational direction (RoD), and the gate 270 includes a first gate portion 271 extending along a circumferential direction (CD), outside of the inner surface of the outer disk 230 in the radial direction (RD) of the outer disk 230, a second gate portion 272 connected to the first gate portion 271 and protruding inwardly from the inner side of the outer disk 230 in the radial direction (RD) along the rotational direction (RoD), and a bolt 278 connecting the gate 270 to a cover portion 250 or the fixed frame 210.

The first gate portion 271 is disposed above the tablet moving surface 231 of the outer disk 230 at a position subsequent to the guide 260 along the rotation direction (RoD). The first gate portion 271 includes an inner plate 271a having a vertical surface and a lower surface 271b spaced apart from the tablet moving surface 231 by a distance G1 corresponding to the height (H) of the tablet (T) and parallel to the tablet moving surface 231. A separation distance (Lgate) from the lower end of the inner plate 271a to the tablet moving surface 231 is formed to be shorter than a shortest distance among the height (H), width (W), and length (Lt) of the tablet.

As illustrated in FIG. 26, when the tablet (T) passes the top dead point (TDP) and while the tablet (T) moves from the inner disk 220 to the outer disk 230, the tablet trying to enter the pocket 243 is blocked by the inner plate 271a with a short separation distance (Lgate), thereby preventing the tablet from entering between the lower surface 271b of the first gate portion 271 and the tablet moving surface 231. After a certain distance in the direction of rotation, the lower end of the inner plate 271a rises to the position of the lower surface 271b, and accordingly, the tablet (T) blocked by the inner plate 271a enters between the lower surface 271b of the first gate portion 271 and the tablet moving surface 231.

The position where the tablet (T) blocked by the inner plate 271a enters, for example, the position where the lower end of the inner plate 271a rises to the position of the lower surface 271b, may be a position where the tablet (T) may protrude and fall from the tablet moving surface 231 of the outer disk 230 if the position of the tablet (T) is not desirable due to the inner plate 271a. For example, a height difference occurs between the outer disk 230 and the inner disk 220, and for example, when the position is beyond the position where the tablet of the outer disk 230 is prevented from falling due to the tablet of the inner disk 220, for example, when the height difference between the inner disk 220 and the outer disk 230 is 1/2 or more of the height (H) or width (W) of the tablet (T); even if the tablet (T) is located at the outermost side of the inner disk 220 due to the height difference, the tablet (T) on the outer disk 230 may not be supported.

Since multiple tablets (T) are transferred at once from the dead top point (TDP), even if the tablets (T) are laid down by the guard 260, the posture of the tablets (T) may change during the transfer process, and in this state, the tablets (T) may be damaged if they try to enter between the lower surface 271b and the tablet moving surface 231.

In the present disclosure, the first gate portion 271 includes an inner plate 271a, and prevents the tablets (T) that pass from the top dead point (TDP) to the outer disk 230 from entering directly under the gate lower surface 271b, thereby preventing the tablets (T) from being caught between the lower surface 271b and the tablet moving surface 231 and being damaged. After the rapid movement is completed, the lower end of the inner plate 271a rises to a predetermined distance, so that the lying tablet (T) naturally enters the lower surface 271b and the tablet moving surface 231 by the centrifugal force caused by the rotation of the outer disk 230 and the inclination of the tablet moving surface 231. In addition, when the tablet (T) is not lying, the tablet is blocked by the inner plate 271a and is prevented from entering the space between the lower surface 271b and the tablet moving surface 231.

At the top dead point (TDP), the position where the inner plate 271a blocks the tablet (T), for example, the distance between the inner plate 271a and the inner surface of the outer disk 230, may be about 1/2 of the width (W) of the tablet (T) in order for the length (L) direction of the tablet (T) to be aligned with and match the circumferential direction. The distance between the inner plate 271a and the inner surface of the outer disk 230 varies depending on the size of the tablet. If the distance exceeds 1/2 of the width, the tablet (T) may not fall from the outer disk 230 due to its own weight even when the longitudinal direction of the tablet (T) coincides with the radial direction.

The second gate portion 272 includes an inner plate 272a and a lower surface 272b spaced apart from the tablet moving surface 231 by a distance G1 corresponding to the height (H) of the tablet (T) and parallel to the tablet moving surface 231. The inner plate 272a is configured as an extension of the inner plate 271a of the first gate portion 271, and the lower surface 272b may also be an extension of the lower surface 271b of the first gate portion 271.

In the second gate portion 272, the inner plate 272a is formed to have a radius of curvature R2 from the center of radius C2 when viewed in a plan view (see FIG. 16). At this time, the radius of curvature R2 is smaller than the distance from the center of rotation (CoD) of the outer disk 230 to the first gate portion 271, so that the second gate portion 272 is configured to protrude from the outer disk 230 along the rotation direction.

As illustrated in FIG. 25, the lower end of the inner plate 272a rises along the inclined tablet moving surface 231, which performs the function of pushing out the tablet (T) that did not enter the space between the lower surface 271b and the tablet moving surface 231 in the second gate portion 272, from the outer disk 230 through the inner plate 272a.

The gate 270 prevents the tablet (T) coming over from entering the space between the lower surface 271b of the gate 270 and the tablet moving surface 231, thereby preventing damage due to jamming, and only allows the laid-down tablet (T) to enter the space between the lower surface 271b of the gate 270 and the tablet moving surface 231, and allows the rest to fall from the outer disk 230 to the upper portion of the inner disk 220, thereby aligning the tablet (T) in a set position without damaging to the tablet.

In this embodiment, the gate 270 is described as having a structure in which the inner plates 271a and 272a are attached, but the inner plates 271a and 272a may also be manufactured as a structure integrated into the gate 270 rather than as a separate structure.

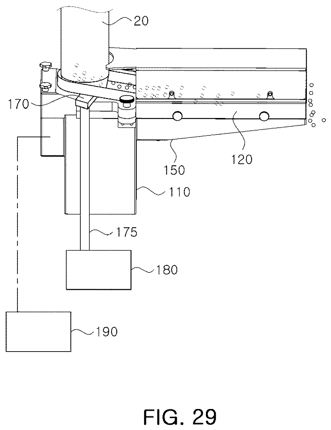

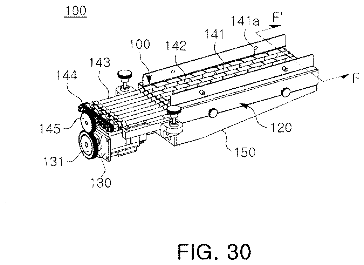

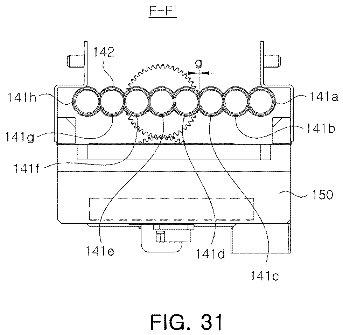

FIGS. 29 to 31 illustrate a vibrating screw supply unit 100. In detail, FIG. 29 illustrates a schematic perspective view of the vibrating screw supply unit 100, FIG. 30 illustrates an exploded perspective view of the vibrating screw supply unit 100, and FIG. 31 illustrates a cross-sectional view taken along line F-F' of FIG. 30.

The vibrating screw supply unit 100 includes a vibration portion 170 connected to a hopper connection pipe 20 and moving tablets supplied therefrom by vibration, a screw moving portion 120 receiving tablets supplied from the vibrating portion 170 and moving the received tablets to a centrifugal feeder, and a pillar portion 110 connected to a device frame 10.

The vibrating portion 170 is positioned higher than the screw moving portion 120 and the pillar portion 110, and includes an inclined surface. The vibrating portion 170 is connected to a vibration generating unit 180 by a vibration transmission column 175 connected to the vibration generating unit 180 disposed below the device frame 10, and moves the tablet (T) along the inclined surface to the screw moving portion 120 due to the vibration transmitted by the vibration generating unit 180.

The vibration generating unit 180 is connected to the aforementioned control unit 600, and the amount of vibration is adjusted according to the amount of the tablet (T) stored on the upper portion of the inner disk 220 of the centrifugal feeder 200, thereby adjusting the amount of the tablet (T) delivered to the screw moving portion 120.

The screw moving portion 120 extends in the direction of the tablet movement, and includes a plurality of screws 141 having protrusions 142 formed spirally, a screw gear 144 formed on the end of the screw 141, a connecting bar 143 connecting the screw gear 144 and the screw 141, a driving motor 130 that provides power to rotate the screw 141, and gears 131 and 145 transmitting power between the driving motor 130 and the screw gear 144.

The screw 141 includes first to eighth screws 141a to 141f, and each of the first to eighth screws 141a to 141f has a spiral protrusion 142 formed thereon. As the screw 141 rotates, a tablet (T) placed on the screw 141 is moved in the screw extension direction, for example, the tablet movement direction, by the spiral protrusion 142.

The screw gears 144 disposed at the ends of the screws 141 are interlocked with each other, and accordingly, the first to eighth screws 141a to 141h rotate in opposite directions to the neighboring screws 141, and accordingly, the spiral protrusions 142 are also formed to rotate in opposite directions to the neighboring screws 141. Since the screws 141 are formed with the protrusions 142, a gap (g) corresponding to at least the height of the protrusions 142 is formed between the screws 141.

A collection unit 150 is disposed below the screw moving portion 120, covers the screw 141 arrangement area and slopes downward toward the pillar portion 110. The collection unit 150 is connected to the vacuum forming portion 190, on the pillar portion 110 side.

The tablets (T) supplied through the hopper connection pipe 20 are supplied to the screw moving portion 120 by inclination and vibration from the vibrating portion 170. In the screw moving portion 120, the tablets (T) supplied from the vibrating portion 170 are moved by the rotation of the screw 141, and fall from the end and fall to the upper portion of the inner disk 220 of the centrifugal feeder 200.

In the process of being supplied from the hopper connection pipe 20, since tablets (T) are supplied in large quantities at once, there are cases where tablets (T) are damaged due to collisions between tablets (T). In the case of the vibrating portion 170, since vibration is transmitted to move the tablets, even if there are broken tablets (T), it is not easy to remove the broken tablets. In an embodiment of the present disclosure, a screw moving portion 120 is additionally disposed in the vibrating portion 170, so that the broken tablet (T) crumbs fall into the collection unit 150 through the gap (g) formed in the screw moving portion 120, and the transfer to the centrifugal feeder 200 is possible by removing the broken tablet (T) crumbs.

In detail, since the spiral protrusion 142 rotates in the screw moving portion 120, it is easy to move the tablets (T), but it is also easy to discharge the crumbs mixed in the tablets (T) downward. In addition, the collection unit 150 is connected to the vacuum forming portion 190, so that fine powder may be sucked out to the vacuum forming portion 190, and crumbs and fragments may also be removed together.

In addition, in the vibrating screw supply unit 100, the supply amount is controlled by the vibration amount of the vibrating portion 170, and the screw moving portion 120 always rotates at a constant speed. The screw moving portion 120 performs the role of discharging fragments while moving, and since the movement by the rotation of the screw 141 is more difficult to transport stably than the movement due to the vibration amount of the vibrating portion 170, the supply amount is controlled through the vibrating portion 170, and the screw moving portion 120 focuses on moving at a constant speed and discharging fragments, thereby having the advantage of being able to remove fragments while controlling the supply amount.

As set forth above, according to some embodiments, a centrifugal feeder in which various tablets may be rapidly supplied in a certain posture and/or position without damage to the tablets through the configurations described above, and a tablet inspection device including the same.

While example embodiments have been illustrated and described above, it will be apparent to those skilled in the art that modifications and variations could be made without departing from the scope of the present disclosure as defined by the appended claims.

## Claims

1. A centrifugal feeder comprising:
an inner disk having tablets supplied thereto and rotating with a central axis thereof inclined relative to a vertical direction;
an outer disk rotating around while surrounding the inner disk and including a tablet moving surface in which the tablets received from the inner disk are moved; and
a pocket guide connected to the outer disk, covering a portion of the tablet moving surface and including a plurality of pockets into which the tablets are received.

2. The centrifugal feeder of claim 1, wherein the pocket guide includes a pocket cover portion covering an outer surface of the outer disk and a protrusion connected to the pocket cover portion and extending to the tablet moving surface to form the plurality of pockets.

3. The centrifugal feeder of claim 2, wherein the pocket guide is configured to be replaceable from the outer disk.

4. The centrifugal feeder of claim 2, wherein the protrusion protrudes from one side connected to the cover portion toward a center of rotation of the outer disk, and partitions neighboring pockets, and
a width of the protrusion decreases in a circumferential direction as approaching the center of rotation.

5. The centrifugal feeder of claim 4, wherein a pitch from a vertex of the protrusion to a vertex of a neighboring protrusion is smaller than twice a maximum width of the tablets supplied to the centrifugal feeder.

6. The centrifugal feeder of claim 4, wherein the protrusion includes a first curved surface connected to a neighboring protrusion and having a first radius of curvature.

7. The centrifugal feeder of claim 6, wherein the protrusion includes a plane extending obliquely from the first curved surface.

8. The centrifugal feeder of claim 6, wherein a vertex of the protrusion is formed as a second curved surface having a second radius of curvature, and the first radius of curvature is larger than the second radius of curvature.

9. The centrifugal feeder of claim 7, wherein the plane is inclined at a first angle (θ) with respect to a radial direction of the pocket guide,
wherein the first angle is greater than 0° and less than or equal to 60°.

10. The centrifugal feeder of claim 9, wherein the first angle is between 20° and 40°.

11. The centrifugal feeder of claim 10, wherein a height of the protrusion is between 4/3 times and 1.5 times the first radius of curvature.

12. The centrifugal feeder of claim 2, wherein the cover portion is formed by bending to cover a portion of a lower surface in addition to the outer surface of the outer disk.

13. The centrifugal feeder of claim 1, further comprising:
a fixed frame surrounding the outer disk; and
a guide connected to the fixed frame, and including a guide surface positioned by a second angle from a top dead point of the inner disk in a direction opposite to a rotational direction of the outer disk when viewed from above, being a portion in which the inner disk and the outer disk come into contact in a radial direction, and protruding from a starting position of a height of the outer disk in a vertical direction to an area of the inner disk in the rotational direction.

14. The centrifugal feeder of claim 13, wherein at the starting position of the guide surface, the outer disk is positioned higher than the inner disk by a first distance,
wherein the first distance is between 1/2 and 9/10 of a width of a target tablet.

15. The centrifugal feeder of claim 13, wherein the guide surface has an angle formed with a horizontal plane and decreasing gradually in the rotational direction.
